# EUROPÄISCHE PATENTSCHRIFT

(11) **EP 3 452 154 B1**
(45) Veröffentlichungstag und Bekanntmachung des Hinweises auf die Patenterteilung: **31.03.2021**
(21) Anmeldenummer: 17725526.2
(22) Anmeldetag: 04.05.2017
(51) Int. Cl.: A61M 16/00, A61B 5/145, A63B 23/18

(54) **VORRICHTUNG ZUR VERBESSERUNG DER KOGNITIVEN UND FUNKTIONSFÄHIGKEITEN MITTELS HYPOXISCHER UND HYPEROXISCHER GASMISCHUNGEN**
DEVICE FOR IMPROVING COGNITIVE AND FUNCTIONAL ABILITIES USING HYPOXIC AND HYPEROXIC GAS MIXTURES
DISPOSITIF D'AMÉLIORATION DES APTITUDES COGNITIVES ET FONCTIONNELLES PAR DES MÉLANGES GAZEUX HYPOXIQUES ET HYPEROXIQUES

(30) Priorität: 06.05.2016 EP 16168560
(43) Veröffentlichungstag der Anmeldung: 13.03.2019
(73) Patentinhaber: Ai Mediq S.A., 1273 Luxembourg (LU)
(72) Erfinder: GLAZACHEV, Oleg S., Moscow 127473 (RU); PLATONENKO, Alexey, 2145 Luxembourg (LU); SPIRINA, Galina, Moscow 107076 (RU); GOLITSYN, Petr, Moscow 119334 (RU); LIKAR, Rudolf, 9020 Klagenfurt (AT); PINTER, Georg, 9020 Klagenfurt (AT)
(74) Vertreter: Jeck, Anton
(86) Internationale Anmeldenummer: PCT/EP2017/060693
(87) Internationale Veröffentlichungsnummer: WO 2017/191275

(56) Entgegenhaltungen:
- EP-A1- 1 721 629
- CN-B- 102 670 326
- DE-U1-202010 009 330
- DE-U1-202012 012 602
- RU-C1- 2 365 384
- US-A1- 2007 221 225

## Beschreibung

Die Erfindung betrifft Vorrichtungen zur Verbesserung der kognitiven und Funktionsfähigkeiten durch Einatmung von hypoxischen und hyperoxischen Gasmischungen, insbesondere mittels eines speziell ausgebildeten Hypoxikators (https://en.wikipedia.org/wiki/Hypoxicator), besonders bevorzugt mittels eines Geräts nach Art eines Reoxy-Geräts oder CellAir One-Geräts.

Ein Hypoxikator ist eine Vorrichtung, welche dazu bestimmt ist, einen Anreiz für die Anpassung des Herz-Kreislauf-Systems eines Individuums durch Atmung reduzierte Sauerstoff-Hypoxie-Luft und Auslösemechanismen der Kompensation bereitzustellen.

Ein Reoxy-Gerät ist ein speziell ausgebildeter Hypoxykator, mittels welchem Patienten während der Behandlung personalisierte Gaben eines sauerstoffreduzierten (Hypoxie) Luft-Gas-Gemisch zuführbar sind. Im Unterschied zu herkömmlichen Hypoxikatoren weist ein speziell ausgebildeter Hypoxykator eine Betriebsweise auf, in welcher intervallweise neben den sauerstoffarmen Gasen sauerstoffangereicherte Gase erzeugt und einer Person zuführbar sind.

Aus der EP 1 721 629 A1 gehen eine Vorrichtung und ein Verfahren zum intermittierenden hypoxischen Training unter Kontrolle des Biofeedbacks hervor. Dabei wird eine Vorrichtung verwendet, welche ausgebildet ist, einer Person abwechselnd eine sauerstoffentreicherte (hypoxische) Luft oder gewöhnliche Luft zuzuführen.

Die DE 20 2012 012602 U1 lehrt eine Vorrichtung zur Durchführung eines Hypoxietrainings, wobei ununterbrochen die Überwachung physiologischer Kenngrößen wie beispielsweise die Sauerstoffkonzentration im Blut durchgeführt wird, und auf Basis einer biologischen Rückkopplung die Intervalle des Trainings eingestellt werden. In den Erholungsphasen wird entweder Normluft oder ein hyperoxisches Gas der Person zugeführt.

Aus der EP 1 721 629 A1 geht eine Vorrichtung zum bereitstellen verschiedener Gasmischungen hervor, wobei eine Membran vorgesehen ist, welche hypoxisches Gas produzieren kann. Weiterhin wird ausgeführt, dass an den Benutzer der Apparatur sowohl hyperoxisches als auch hypoxisches Gas geleitet werden kann.

Das nicht beanspruchte Verfahren zur Verbesserung von kognitiven und Funktionsfähigkeiten von Personen mittels Veränderung des Blutsaturationsgrades mit Sauerstoff oder mittels Veränderung der Sauerstoffkonzentration in der eingeatmeten Gasmischung umfasst:
a) die (intermittierende) Einatmung von hypoxischen und hyperoxischen Gasmischungen über eine Gesichtsmaske,
b) die Berechnung von Behandlungsparametern aufgrund einer individuellen Reaktion der Person auf die Veränderung der Sauerstoffkonzentration in der eingeatmeten Gasmischung, wobei die Sauerstoffkonzentration eines Pulsoximeters gemessen wird,
c) die kontinuierliche Überwachung der arteriellen Sauerstoffsättigung und Herzfrequenz mit Hilfe eines Pulsoximeters.

Die Vorrichtung zur Verwendung in den oben genannten Verfahren ist fähig, Atmungsgasmischungen mit einem niedrigeren (9-21 Vol. %) und einem erhöhten (21-95 Vol. %) Sauerstoffanteil zu generieren, indem die Freiluft in Stickstoff und Sauerstoff aufgeteilt und nachfolgend gemäß einem berechneten Verhältnis vermengt wird, um diese Gasmischung nachfolgend über eine Gesichtsmaske der Person zuzuführen. Dabei wird sie angewendet, um kognitive und Funktionsfähigkeiten der Person mittels Veränderung des Blutsaturationsgrades mit Sauerstoff o d er mittels Veränderung der Sauerstoffkonzentration in der eingeatmeten Gasmischung zu verbessern und umfasst die intermittierende Einatmung von hypoxischen und hyperoxischen Gasmischungen über eine Gesichtsmaske, die mittels eines Schlauchs mit der Vorrichtung für hypoxischen und hyperoxischen Gasmischungen verbunden ist.

Die hypoxische Gasmischung zur Verwendung in der erfindungsgemäßen Vorrichtung und dem erfindungsgemäßen Verfahren ist eine Gasmischung mit einem Sauerstoffanteil zwischen 21 und 9 Vol. %, und die hyperoxische Gasmischung ist eine Gasmischung mit einem Sauerstoffanteil zwischen 21 und 95 Vol. %.

Die Dauer einer Behandlung (Sitzung) mit der hypoxischen Gasmischung des hypoxischen Intervalls wird aufgrund einer individuellen Reaktion der Person auf die Verminderung der Sauerstoffkonzentration in der eingeatmeten Gasmischung berechnet. Dies kann aufgrund des Verminderungsgrades der Sauerstoffsättigung des Blutes geschehen, wobei die Dauer maximal 1200 Sekunden umfasst.

Die Dauer der Behandlung mit der hyperoxischen Gasmischung des hyperoxischen Intervalls wird aufgrund einer individuellen Reaktion der Person auf die Erhöhung der Sauerstoffkonzentration in der eingeatmeten Gasmischung berechnet, und zwar aufgrund des Grades der Wiederherstellung der Sauerstoffsättigung des Blutes, wobei die ursprünglichen Werte zur Anfangszeit der Zuführung der hypoxischen Gasmischung wiederhergestellt werden. Bei einem schnellen Anstieg der Sauerstoffkonzentration des Blutes, insbesondere einer schnellen Sauerstoffsättigung des Blutes nach einer Behandlung mit der hyperoxischen Gasmischung kann die hyperoxische Gasmischung insgesamt mit einer geringen Sauerstoffkonzentration eingestellt werden und/oder die Behandlungsdauer mit der hyperoxischen Gasmischung verkürzt werden. Steigt die Sauerstoffkonzentration der Person nach einer Behandlung mit der hyperoxischen Gasmischung langsam an, kann die Sauerstoffkonzentration der hyperoxischen Gasmischung erhöht werden und/oder die Behandlung mit der hyperoxischen Gasmischung verlängert werden.

Zur Bestimmung einer schnellen bzw. langsamen Sättigung des Blutes wird ein Beobachtungszeitrahmen eingestellt, in welchem die Regeneration der Sauerstoffsättigung nach einer Behandlung mit einer Standard hypoxischen Gasmischung beobachtet wird. Die Standard hypoxische Gasmischung weist vorzugsweise eine Sauerstoffkonzentration von 12 Vol. % auf. Vorzugsweise beträgt der Beobachtungszeitrahmen 3 min. Besonders bevorzugt weist die Standard hyperoxische Gasmischung einen Sauerstoffanteil von Vol. 30 % auf. Wird eine Sauerstoffsättigung des Blutes in dem Beobachtungszeitrahmen nicht erreicht, kann ein langsamer Anstieg der Sauerstoffkonzentration des Blutes, insbesondere eine langsame Sauerstoffsättigung des Blutes vorliegen.

Zum Erzeugen der hyperoxischen und hypooxischen Gasmischungen kann die erfindungsgemäße Vorrichtung eine Kammer aufweisen, in welcher eine Membran zur Gastrennung, insbesondere eine Tsiolit-Membran vorgesehen ist. Durch das teilweise hindurchtreten von Bestandteilen der Luft durch die Membran werden zwei Gasströme gebildet, ein Sauerstoff angereicherter erster Gasstrom und ein Sauerstoff abgereicherter zweiter Gasstrom. Der erste Strom weist einen Anteil von 50 +/-10 Vol. % Sauerstoff auf. Der zweite Strom weist einen Anteil von 85 +/- 3 Vol. % Stickstoff auf. Vorzugsweise ist der zweite Gasstrom so gebildet, das er bei Rekombination mit dem ersten Gaststrom gewöhnliche Luft bildet. Beide Gasströme können jedoch auch mit zusätzlichem Sauerstoff bzw. Stickstoff über entsprechende Gasquellen wie Gasflaschen weiter angereichert werden.

Der zweite Gasstrom, insbesondere der Stickstoff, wird durch einen Gasmischer in einen ersten Sammel-Beutel geführt. Der erste Gasstrom, insbesondere der Sauerstoff, wird auch in einen zweiten Sammel-Beutel zugeführt und über ein Proportionalventil zu dem Gasmischer geleitet.

Die Menge an Sauerstoff (Strom), welche dem Strickstoffstrom vor/bei der Abgabe beigemengt wird, wird so bestimmt, dass der Beutel mit Atemgasgemisch auf eine vorbestimmte, eingestellte, veränderbare Konzentration gebracht wird, und zwar jeweils sowohl für hypoxische und hyperoxische Intervalle. Gase werden an dem Einlass zu dem Gasmischer und die Ausgabe von der Sicherungstasche gesteuert.

Die Sitzung(en) dauert(en) jeweils 10 bis 90 Minuten. Die Anzahl der Intervalle wird durch die voreingestellte Dauer der Sitzung bestimmt. Die Anzahl der Sitzungen kann zwischen 5 und 25 variieren.

Die Vorrichtung kann eine Gesichtsmaske, bevorzugt eine Nasen-Mund-Maske, umfassen. Das Pulsoximeter kann einen Finger- und/oder Ohrsensor umfassen.

Die kognitiven Funktionen werden mittels Demenz-Detektions-Test (DemTec) und Clock-Drawing-Test-of Sunderland (CDT) bewertet; aber nicht beschränkt darauf; die funktionale Übungskapazität wird mittels eines 6-Minuten-Walk-Tests (6MWT) ermittelt; aber nicht beschränkt darauf.

Die (intermittierende) Einatmung von hypoxischen und/oder hyperoxischen Gasmischungen mittels eines Geräts nach Art eines Reoxygeräts führt zu verbesserten kognitiven Funktionen und/oder verbesserter funktionaler Übungskapazität bei den untersuchten Patienten.

In der EP 1 721 629 A1 ist kein IHHT Training im Sinne der vorliegenden Erfindung offenbart. Insbesondere die Sauerstoffkonzentrationen der hyperoxischen Gasmischung, das Vorsehen einer hypoxischen Gasmischung, der Anwendungsintervalle der einzelnen Gasmischungen sowie der Anzahl an Intervallwiederholungen, welche zu einer Steigerung kognitiver Fähigkeiten führt, gehen aus dieser Druckschrift nicht hervor.

Im Unterschied dazu ist die erfindungsgemäße Vorrichtung ausgebildet, sowohl hypoxische Gasmischungen als auch hyperoxische Gasmischungen bereitzustellen. Diese werden einer Person nach dem erfindungsgemäßen Verfahren abwechselnd in einem Intervalltraining (IHHT) zugeführt.

Die Erfindung wird anhand des folgenden Anwendungsbeispiels näher beschrieben:

### Beispiel 1

Burtcher M. at al (Austria) führten eine randomisierte, geschichtete (abgestufte) placebokontrollierte Doppelblindstudie mit 41 beteiligten Patienten im Alter von 64 bis 92 Jahren durch. Sie wurden beliebig auf die Interventionsgruppe und die Kontrollgruppe verteilt. Beide Gruppen absolvierten dasselbe multimodale Training; aber während die Interventionsgruppe zusätzlich parallel dazu Behandlungen mittels Einatmung von hypoxischen und hyperoxischen Gasmischungen (nachfolgend IHHT) erhielt, erhielt die Kontrollgruppe hingegen eine normoxische Luftmischung. Änderungen der kognitiven Leistungsfähigkeit wurden mit dem Demenz-Detektions-Test (DemTect) und dem Clock-Drawing-Test-of Sunderland (CDT) bewertet. Veränderungen der funktionellen Übungskapazität wurden mittels des Total Distance of 6-Minute-Walk-Tests (6MWT) gemessen.

Für die Hypoxiegruppe dauerte jede IHHT Sitzung bis zu einer Stunde. Die IHHT Sitzungsparameter (maximale Dauer der Hypoxie-Periode, maximale Dauer der Hyperoxie Periode, hypoxische 02 Konzentration) wurden individuell berechnet, basierend auf den Ergebnissen des hypoxischen Tests- HT (02 Konzentration - 12 %, Dauer der hypoxischen Periode - maximum 10 min, hyperoxische Periode - maximum 3 min).

Nach diesem Test wurden die Beteiligten aus der hypoxischen Gruppe einer wiederholten 4 - 7 Minuten langen Einwirkung mit hypoxischer Gasmischunug (10 - 14 %) und anschließender 2 - 4 Minuten langer Einwirkung mit hyperoxischer Gasmischung (30 %) durch eine Gesichtsmaske an jedem Behandlungstag ausgesetzt. Jede Sitzung dauerte 30 bis 40 Minuten und umfasste 4 bis 8 Hypoxie-Hyperoxie-Zyklen je nach der jeweiligen individuellen Reaktion und dem Befinden.

Die normoxische Gruppe (Kontrollgruppe) führte das Programm in derselben Weise durch, wobei sie nur normoxische Luft atmete - Scheinbehandlung.

Die Forscher führten IHHT-Sitzungen unter Einsatz des ReOxy-Geräts für Atemtherapie durch. Dieses Gerät versorgte die Beteiligten mit Gasmischungen (10 - 30 %), wobei gleichzeitig die arterielle Sauerstoffsättigung (SaO2) überwacht wurde. Der SaO2-Anteil sowie die Herzrate wurden mit Hilfe eines Pulsoximeters gemessen, welches alle Messdaten an das Gerät sendete, ohne dass die Testpersonen diese sehen konnten.

Während des ganzen Aufenthalts an der geriatrischen Tagesklinik wurden insgesamt 12 - 15 hypoxische oder Scheinbehandlungen zwei- bis dreimal wöchentlich innerhalb von 5 bis 7 Wochen für beide Gruppen durchgeführt.

Bezüglich der kognitiven Tests mittels DemTect zeigte die Kontrollgruppe eine hohe signifikante Zunahme nach der IHHT kombiniert mit dem MIT (+16.7 %), während die Kontrollgruppe, welche nur MIT ausführte, keine Zunahme aufwies (-0.39 %). Das CDT zeigte ähnliche Ergebnisse mit einer signifikanten Zunahme der Interventionsgruppe (+10.7 %) wohingegen der Score der Kontrollgruppe sogar leicht abnahm (-8 %). Bezüglich der funktionellen Übungskapazität zeigten beide Gruppen eine Zunahme der Gesamtdistanz im 6MWT; aber bei der Interventionsgruppe war die Zunahme signifikant höher als in der Kontrollgruppe (+24.1 % vs. +410.8 %).

### Detaillierte Beschreibung

Mehr als 46 Mio. Menschen leiden heutzutage an Demenz aufgrund von schweren wirtschaftlichen und finanziellen Belastungen. Diese Zahl wird gegen 2050 höchstwahrscheinlich noch auf 131,5 Mio. dramatisch steigen. Es besteht immer noch ein großer Bedarf an neuen Strategien zur Prävention und Behandlung von Demenz.

Eine solche Strategie kann IHHT sein. IHHT erwies sich als ein nicht-invasives, einfach anwendbares und sicheres Verfahren, dem wiederholte Einwirkung mit einer Gasmischung mit reduziertem Sauerstoffanteil zugrunde liegt, wobei diese Einwirkung durch Erholungsphasen von Zeit zu Zeit unterbrochen wird. Diese Erholungsphasen können entweder normoxisch (20 - 21 Vol. % 02, IHT) oder hyperoxische (21 - 95 Vol. % 02, IHHT) sein. Die wiederholte Einwirkung der Gasmischung mit reduziertem Sauerstoffanteil verursacht eine nur mäßige Belastung gefolgt von wohltuenden Anpassungen. Es wurde von Burtscher M. Katayama und K. gezeigt, dass sich die IHT-Übungstoleranz bei Patienten erhöht, die an chronischen Krankheiten, wie Herz-Kreislauferkrankungen, chronischer Lungenkrankheit oder Stoffwechselerkrankungen, leiden. Es ist auch nachgewiesen, dass IHT oxidativen und nitrosativen Stress verhindern kann, gegen neurodegenerative Veränderungen schützt und sogar kognitive Funktionen bei experimenteller Alzheimer-Krankheit bei Ratten verbessert. Unserem Wissen nach waren Schega L. et al die ersten, die Auswirkungen von IHT auf die kognitive Leistungsfähigkeit bei Senioren zwischen 60 und 70 Jahren untersuchten. In ihrer Pilotstudie erhöhte ein Kraftausdauertrainingsprogramm die kognitiven Funktionen, und die zusätzliche IHT ergänzte diesen positiven Effekt der Übungen in Bezug auf die kognitive Leistungsfähigkeit und Lebensqualität.

Es wird eine neue Form der hypoxischen Einwirkung vorgeschlagen, die als Intermittierendes hypoxisches/hyperoxisches Training (IHHT) bezeichnet wird. Das IHHT ist durch noch mehr positive Wirkungen als die herkömmliche IHT gekennzeichnet. Die normoxischen Perioden werden durch mäßige hyperoxische Perioden mit 30-40 % 02 ersetzt. Das führt zur schnelleren Erholung von der Sauerstoff-Entsättigung. Es wird behauptet, dass die hypoxische-hyperoxische Behandlung in einer Studie mit männlichen Wistar-Ratten einen besseren und schnelleren membranstabilisierenden Effekt in Herz-, Leber- und Hirnzellen im Vergleich zu IHT ergibt. Eine aktuelle Studie hat gezeigt, dass die IHHT die Trainingsleistung bei Sportlern mit Übertrainingssyndrom verbessert hat. Die Fallstudie von Susta et al. ermittelte eine deutliche Verbesserung der kardiopulmonalen Effizienz (Herz-Lungen-Effizienz) und Lactatentfernung nach 3 Wochen langer täglicher IHHT. Glazachev zeigte, dass IHHT die Übungstoleranz, die aerobe Kapazität und das kardiometabolische Profil bei Patienten mit NYHA II-III ohne jegliche Übungen verbessern kann. Keine dieser Studien berichtet von unerwünschten Nebenwirkungen. IHHT scheint sogar die positiven Ergebnisse von IHT zu überschreiten.

Das im Rahmen der Forschungen von Burtcher eingesetzte ReOxy-Gerät ist unter www.reoxy.lu näher beschrieben. Dieses Gerät verwendet die Self Regulated Treatment (SRT) Technologie. Die SRT-Technology beruht auf dem Biofeedback-Prinzip, wobei die Körperfunktionen des Patienten, die während der gesamten Behandlungsdauer überwacht werden, die Behandlungsparameter bestimmen. Die SRT-Technologie basiert auf einer hochentwickelten Software, die ständig Daten des eingebauten Pulsoximeters ausliest und analysiert, um die Zusammensetzung des verabreichten Luftgemisches und dessen zeitliche Regulierung anzupassen. Letztgenannte basieren auf Veränderungen der Vitalfunktionen des Patienten (z. B. Sauerstoffsättigung des Blutes (SpO2) und Herzschlagfrequenz) (www.reoxy.lu).

Die vorliegende Erfindung betrifft insbesondere eine Vorrichtung zu Bereitstellung von hypoxisch/hyperoxischer Gasmischungen, mit Mittel zur kontinuierlichen Messung der arteriellen Sauerstoffsättigung und/oder Herzfrequenz sowie zur Ermittlung von individuellen Parametern aufgrund einer individuellen Reaktion der Person auf die körperliche Einwirkung der hypoxischen/hyperoxischen Gasmischungen, wobei die Vorrichtung ausgebildet ist, Gasmischungen mit einem niedrigen und einem erhöhten Sauerstoffanteil zu generieren, indem die Freiluft in Stickstoff und Sauerstoff aufgeteilt und nachfolgend in Abhängigkeit von der individuellen Reaktion gemäß einem berechneten Verhältnis vermengt wird, wobei die Vorrichtung zum intermittierenden hypoxisch/hyperoxischem Training (IHHT) von Personen anwendbar ist, denen die Gasmischungen mittels einer Apparatur, welche mit der Vorrichtung für hypoxische und hyperoxische Luft verbunden ist, zugeführt wird. Erfindungsgemäß ist es vorgesehen, dass der niedrige Sauerstoffanteil 8 % - 20 Vol % und der erhöhte Sauerstoffanteil 21 % - 95 Vol % umfasst.

Nach einer Weiterbildung der Erfindung ist es vorgesehen, dass die hypoxische Gasmischung 10 % - 16 % und die hyperoxische Luftmischung 30 % - 40 % Sauerstoff umfasst.

Vorzugsweise ist es vorgesehen, dass eine Gesichtsmaske vorgesehen ist, mittels welcher die hypoxische/hyperoxische Gasmischung inhalierbar ist und/oder dass die Vorrichtung ein Pulsoximeters aufweist, mittels welchem die arterielle Sauerstoffsättigung und/oder Herzfrequenz bestimmbar ist, wobei das Pulsoximeter einen Finger- und/oder Ohrsensor umfassen kann, mittels welchem die arterielle Sauerstoffsättigung und/oder Herzfrequenz erfassbar ist.

Bevorzugter Weise arbeitet die Vorrichtung nach dem Biofeedbackprinzip. Weiterhin betrifft die vorliegende Erfindung ein Verfahren zur Bereitstellung von intermittierendem hypoxischem/hyperoxischem Training (IHHT), umfassend die Bereitstellung von hyperoxisch/hypoxischer Luft an eine Person unter Verwendung der Vorrichtung gemäß einem der Ansprüche 1 bis 10, wobei (a) die arterielle Sauerstoffsättigung und/oder Herzfrequenz kontinuierlich gemessen werden; (b) individuelle Parameter aufgrund einer individuellen Reaktion der Person ermittelt werden; (c1) die Sauerstoffkonzentration in der zugeführten hypoxisch/hyperoxischen Luft entsprechend der in (b) ermittelten Werte angepasst wird und/oder (c2) der Blutsaturationsgrad mit Sauerstoff verändert wird.

Vorzugsweise wird die Dauer des hypoxischen Intervalls so berechnet, dass die Wiederherstellung des Blutsaturationsgrades mit Sauerstoff auf die Saturationswerte am Anfang des vorhergehenden Zufuhrintervalls sichergestellt ist, wobei das Training während einer ausreichenden Anzahl von Trainingssitzungen wiederholt werden kann, bevorzugt zwischen 5 und 25 mal.

Besonders bevorzugt kann die Dauer des hypoxischen Intervalls maximal 1200 Sekunden betragen, wobei das IHHT-Training zu verbesserten kognitiven Funktionen und/oder verbesserter funktionaler Kapazität bei körperlichen Beanspruchungen führen kann.

Vorzugsweise werden die kognitiven Fähigkeiten mit Hilfe von genormten Tests ausgewertet, einschließlich des DemTect-Tests und des Clock-Drawing-Tests von Sunderland (CDT), aber nicht beschränkt darauf, wobei die verbesserte funktionale Kapazität bei körperlichen Beanspruchungen mit Hilfe von genormten Funktionstests ausgewertet wird, einschließlich des 6-Minute-Walk-Tests (6MWT), aber nicht beschränkt darauf.

Besonders bevorzugt kann die hypoxische Luftmischung 10 % - 16 % und die hyperoxische Luftmischung 30 % - 40 % umfassen.

Weiterhin wird beschrieben ein Verfahren zum Steuern des Betriebs der erfindungsgemäßen Vorrichtung, bei welchem die Vorrichtung nacheinander wiederholt in einem ersten Modus, in welchem sie hypoxisches Gas erzeugt und in einem zweiten Modus, in welchem sie hyperoxisches Gas erzeugt, betrieben wird, wobei eine Einrichtung der Vorrichtung die arterielle Sauerstoffsättigung und/oder Herzfrequenz einer Person misst und basierend auf den Messwerten die Dauer des Betriebs in dem ersten Modus dynamisch anpasst und einstellt.

Wie beschrieben kann eine Rückkoppelung zwischen der gemessenen Sauerstoffsättigung und/oder Herzfrequenz einer Person und der Dauer der Phase, in welcher hypoxisches Gas bzw. der Phase in welcher hyperoxisches Gas der Person zugeführt wird, dynamisch angepasst und bei jeder Wiederholung der einzelnen Phasen neu eingestellt werden.

Besonders bevorzugt, kann die eine Anpassung bzw. Einstellung der Dauer der Phase, also eine Verlängerung oder Verkürzung der Phase auch dann basierend Sauerstoffsättigung und/oder Herzfrequenz eingestellt werden, wenn die Phase bereits begonnen hat.

Vorzugsweise wird basierend auf der Sauerstoffsättigung und/oder Herzfrequenz der Person auch die Dauer des zweiten Modus eingestellt.

Besonders bevorzugt wird die Vorrichtung in dem ersten Modus mit einer Sauerstoffsättigung von 10-16 Vol. % und in dem zweiten Modus mit einer Sauerstoffsättigung von 30- 40 Vol. % und noch mehr bevorzugt mit 33-37 Vol. % in dem zweiten Modus betrieben.

Erfindungsgemäß sind folgende Definitionen gegeben:
- dass die individuelle Reaktion eine individuelle hypotoxische Reaktion und eine individuelle hypertoxische Reaktion aufweisen kann,
- dass die individuelle hypoxische Reaktion der Person den spezifischen Verminderungsgrad der Sauerstoffsättigung des Blutes aufgrund Verminderung der Sauerstoffkonzentration in der eingeatmeten Gasmischung, insbesondere der hypoxischen Gasmischung aufweisen kann, und
- dass die individuelle hyperoxische Reaktion der Person den spezifischen Grades der Wiederherstellung der Sauerstoffsättigung des Blutes aufgrund der Erhöhung der Sauerstoffkonzentration in der eingeatmeten Gasmischung, insbesondere der hyperoxischen Gasmischung aufweisen kann.

Die erfindungsgemäße Vorrichtung und das beschriebene Verfahren sind ausgebildet bei einem gesunden Menschen, welcher also keine kognitive Mängel aufweist, im Rahmen eines Trainings, insbesondere eine leistungssteigernden Trainings, eine Steigerung seiner kognitiven Fähigkeiten zu ermöglichen.

### Referenzen

1. Basovich SN. Trends in the use of preconditioning to hypoxia for early prevention of future life diseases. Biosci Trends 2013;7:23-32.
2. Burtscher M, Gatterer H, Szubski C, Pierantozzi E, Faulhaber M. Effects of interval hypoxia on exercise tolerance: special focus on patients with CAD or COPD. Sleep Breath. 2009 Aug 18 PubMed PMID: 19688232.
3. Burtscher M, Pachinger O, Ehrenbourg let al (2004) Intermittent hypoxia increases exercise tolerance in elderly men with and without coronary artery disease. Int J Cardiol 96:247-254
4. Korkushko O, Asanov E, Shatilo V, Makovskaya L. Efficeincy of interval normobaric hypoxische training in elderly subjects. Problems of aging and long living. 2004, 13, Vol. 2, 155-161
5. Lei Xi, Serebrovskaya TV. Intermittent hypoxia: from molecular mechanisms to clinical applications. Nova science publishers. 2009.
6. Ischuk V, Shatilov. Intermittent normobaric hypoxia trainings recommendation for elderly ischemic heart disease ptients. Krovoobig ta Gemostaz. 2007, Vol 1: 49-53
7. Ishchuk V. (2007). Safety and efficacy of intermittent normobaric hypoxia training in elderly patients with ischemic heart disease. J. Ukrainian Acad. Med. Sci.13:374-384.
8. Lyamina NP, Lyamina SV, Senchiknin VN, Mallet RT, Downey HF, Manukhina EB. Normobaric hypoxia conditioning reduces blood pressure and normalizes nitric oxide synthesis in patients with arterial hypertension. J Hypertens 2011:29:226572
9. Schega L., Peter B, Törpel A, Mutschler H, Isermann B, Hamacher D. Effects of intermittent hypoxia on cognitive performance and quality of life in elderly adults: a pilot study. Gerontology. 2013; 59(4):316-23.
10. Shatilo VB, Korkushko OV, Ischuk VA, Downey HF, Serebrovskaya TV. Effects of intermittent hypoxia training on exercise performance, hemodynamics, and ventilation in healthy adults. High Alt Med Biol. 2008 Spring;9(1):43-52. PubMed PMID: 18331220.
11. EP 1 721 629 A1

## Patentansprüche

1. Vorrichtung zur Bereitstellung von hypoxischen/hyperoxischen Gasmischungen mit Mitteln zur kontinuierlichen Messung der arteriellen Sauerstoffsättigung und/oder Herzfrequenz sowie zur Ermittlung von individuellen Parametern der Einwirkung von hypoxischen/hyperoxischen Gasmischungen, welche aufgrund einer individuellen Reaktion der Person, durch Atemmischungen mit einem niedrigen und einem erhöhten Sauerstoffanteil generierbar sind, die zum intermittierenden hypoxisch/hyperoxischem Training (IHHT) anwendbar ist, bei dem die Gasmischungen mittels einer Apparatur, welche mit der Vorrichtung für hypoxische und hyperoxische Luft verbunden ist, zuführbar ist, wobei der niedrige Sauerstoffanteil 8-20 Vol. % und der erhöhte Sauerstoffanteil 21-95 Vol. % umfasst
**dadurch gekennzeichnet,**
**dass** die Atemmischungen mit dem niedrigen und einem erhöhten Sauerstoffanteil generierbar sind, indem die Freiluft in Stickstoff und Sauerstoff aufgeteilt und nachfolgend gemäß einem definierten Verhältnis vermengt werden.

2. Vorrichtung nach Anspruch 1,
wobei die hypoxische zum IHHT benutzte Gasmischung 10-16 Vol. % Sauerstoff und die hyperoxische zum IHHT benutzte Gasmischung 21-40 Vol. % Sauerstoff enthält.

3. Vorrichtung gemäß einem der Ansprüche 1 oder 2,
wobei die hypoxische/hyperoxische Gasmischung mittels einer Gesichtsmaske inhalierbar sind.

4. Vorrichtung gemäß einem der Ansprüche 1 bis 3,
wobei arterielle Sauerstoffsättigung und Herzfrequenz mittels eines Pulsoxymetersmessbar sind.

5. Vorrichtung nach Anspruch 4,
wobei das Pulsoximeter einen Finger-und/oder Ohrsensor umfasst.

6. Vorrichtung gemäß einem der Ansprüche 1 bis 5,
wobei die Vorrichtung gemäß dem Biofeedbackprinzip funktioniert.

## Claims

1. Device for providing hypoxic / hyperoxic gas mixtures comprising means for continuous measurement of arterial oxygen saturation and / or heart rate as well as for determining individual parameters of the effect of hypoxic / hyperoxic gas mixtures which gas mixtures are, due to an individual reaction of the person, generated by breathing-mixtures with a low and an increased proportion of oxygen, which can be used for intermittent hypoxic / hyperoxic training (IHHT), in which the gas mixtures are suppliable by means of an apparatus that is connected to the device for hypoxic and hyperoxic air, wherein the low oxygen concentration is 8-20 % Vol. and the increased oxygen concentration is 21-95 % Vol.
**characterized in,**
**that** the breathing mixtures with the low and an increased proportion of oxygen are generated by dividing the open air into nitrogen and oxygen and then mixing them according to a defined ratio.

2. Device according to claim 1,
wherein the hypoxic gas mixture used for IHHT contains 10-16 % Vol. of oxygen and the hyperoxic gas mixture used for IHHT contains 21-40 % Vol. oxygen.

3. Device according to one of claims 1 or 2,
wherein the hypoxic / hyperoxic gas mixture can be inhaled using a face mask.

4. Device according to one of claims 1 to 3,
wherein arterial oxygen saturation and heart rate can be measured using a pulse oximeter.

5. Apparatus according to claim 4,
wherein the pulse oximeter comprises a finger and / or ear sensor.

6. Device according to one of claims 1 to 5,
wherein the device functions according to the biofeedback principle.

## Revendications

1. Dispositif pour la fourniture de mélanges gazeux hypoxiques/hyperoxiques avec des moyens de la mesuration continue de la saturation en oxygène artérielle et/ou la fréquence du cœur et aussi pour la détermination des paramètres individuels de l'action des mélanges gazeux hypoxiques/hyperoxiques qui sont générés suite à une réaction individuelle de la personne par des mélanges de respiration avec une proportion en oxygène inférieure ou supérieure qui est applicable par un entraînement par intermittence hypoxique/hyperoxique (IHHT) où les mélanges gazeux sont acheminés par le biais d'un appareil qui est relié à l'air hypoxique/hyperoxique où la proportion en oxygène inférieure s'élève à 8-20 Vol. % et la proportion en oxygène supérieure à 21-95 Vol. %,
Est caractérisé de sorte que
Les mélanges de respiration sont générés avec la proportion en oxygène inférieure et une proportion en oxygène supérieure où l'air extérieure est réparti en azote et en oxygène et est mélangé par la suite selon un rapport défini.

2. Dispositif selon la revendication 1 où le mélange gazeux hypoxique utilisé dans l'IHHT contient 10-16 Vol. % d'oxygène et le mélange gazeux hyperoxique utilisé dans l'IHHT contient 21-40 Vol. % d'oxygène.

3. Dispositif selon l'une des revendications 1 ou 2 où le mélange gazeux hypoxique/hyperoxique est inhalé grâce à un masque de visage.

4. Dispositif selon l'une des revendications 1 jusqu'à 3 où la saturation en oxygène et la fréquence du cœur sont mesurés par le biais d'un oxymètre de pouls.

5. Dispositif selon la revendication 4 où l'oxymètre de pouls se compose d'un capteur biométrique et/ou d'un capteur à ampoule.

6. Dispositif selon l'une des revendications 1 jusqu'à 5 où le dispositif fonctionne selon un principe de rétroaction biologique.
